Europäisches Patentamt

European Patent Office

Office européen des brevets

(19)

(11) Publication number: **0 096 117**
**B1**

(12) **EUROPEAN PATENT SPECIFICATION**

(45) Date of publication of patent specification: **02.09.87**

(51) Int. Cl.⁴: **G 01 N 27/40,** G 01 N 27/28

(21) Application number: **82200695.3**

(22) Date of filing: **07.06.82**

(54) **Analyzer for chemical oxidizing or reducing agents.**

(43) Date of publication of application:
**21.12.83 Bulletin 83/51**

(45) Publication of the grant of the patent:
**02.09.87 Bulletin 87/36**

(84) Designated Contracting States:
**DE FR GB IT NL**

(56) References cited:
**US-A-3 421 989**
**US-A-3 703 457**
**US-A-4 049 503**
**US-A-4 310 400**

(73) Proprietor: **THE DOW CHEMICAL COMPANY**
**2030 Dow Center Abbott Road P.O. Box 1967**
**Midland, MI 48640 (US)**

(72) Inventor: **Wolcott, Duane Kent**
**3406 Morning Glory**
**Baton Rouge Louisiana 70808 (US)**
Inventor: **Carraway, John Biddle, Jr.**
**4548 Woodlot**
**Orlando Florida 32811 (US)**
Inventor: **Pulliam, Leroy**
**6852 E. Caprice Avenue**
**Baton Rouge Louisiana 70811 (US)**

(74) Representative: **Urbanus, Henricus Maria, Ir.**
**et al**
**c/o Vereenigde Octrooibureaux Nieuwe**
**Parklaan 107**
**NL-2587 BP 's-Gravenhage (NL)**

## Description

This invention relates to the quantitative determination of chemical oxidizing or reducing agents in a gaseous environment. More particularly, the invention relates to the determination of chemical oxidizing or reducing agents by means of a novel type of sensor or detector which includes an ion-exchange membrane.

In the current state of the art, the electrodes forming a part of the electrochemical cell or couple in analyzers are separated from each other by means of a porous layer. The porous layer permits a restricted flow of electrolyte, thereby completing the electrochemical couple between the electrodes in the sensor portion of the analyzers. A serious disadvantage of the porous layer is that it permits a substantial diffusion of sample throughout the electrolyte between the electrodes. As a result, the recovery time necessary to stabilize known analyzers between tests is relatively long, and the only known way to reduce the recovery time is to provide special means of renewing the electrolyte in the analyzers. These special means are not entirely satisfactory in that they are cumbersome, costly, and require frequent and extensive maintenance. The present invention provides an analyzer which is not subject to the limitations of lengthy recovery time or the necessity of providing auxiliary means for restabilizing the electrode system.

A second serious disadvantage of the porous layer is that it is non-selective. The porous layer permits the electrodes to be exposed to all constituents of the fluid being monitored. The result is the possibility of contamination or poisoning of the electrodes, and of direct interference in the quantitative measurement by substances present in the sample other than those being determined. The present invention provides a means which, in combination with the electrodes, substantially eliminates the possibility of contamination or poisoning of the electrodes and of interference by extraneous components of the environment being tested.

It is to be remarked, that from US—A 4,310,400 a thin layer electrode and cell is known for investigating amounts of liquids, which electrode comprises an electroconductive element and an ion exchange membrane in spaced relation to said element, said membrane being positioned in relation to said element in such a manner so as to be capable of retaining a thin layer of investigate liquid between said element and said membrane, and means to admit said investigate liquid between said membrane and said electroconductive element.

The present invention concerns however an instrument for analyzing gaseous components.

The present invention is concerned with an instrument (10) for quantitatively determining the concentration of chemical oxidizing or reducing agents in a gaseous environment, comprising a first electrode (9) exposed to said gaseous environment, a second electrode (3) in contact with an electrolyte solution (4) and means for measuring a flow of electrical current between said first (9) and said second electrode (3).

In US—A 4,049,503 an electrochemical gas detector having these features is described.

The present invention is characterized in that an ion-exchange membrane (6) adapted to contain said electrolyte solution is present, said ion-exchange membrane (6) separating said electrolyte solution (14) and second electrode (3) from said first electrode (9), said first electrode (9) being in electrical and physical contact with said ion-exchange membrane (6).

The choice of membrane depends upon the chemical species being quantitatively determined. If an oxidizing species such as a halogen is to be determined, a cation-exchange membrane is chosen to permit a flow of positive ions from the electrolyte through the membrane to the first electrode. If a reducing species such as hydrogen, carbon monoxide or hydrogen sulfide is to be determined, an anion-exchange membrane may be used to permit a flow of negative ions from the electrolyte through the membrane to the first electrode.

The function of the instrument may be classified as electrochemical in nature. If the first and second electrodes are made of dissimilar metals, the first electrode is characterized as being catalytic with respect to the oxidizing or reducing agent being determined but chemically inert to the agent and fluid environment in which it is positioned, and the second electrode is characterized as being chemically reactive in the electrolyte solution and being consumed by the electrochemical reaction taking place during determination of the agent. If the first and second electrodes are made of the same metal, they are characterized as both being catalytic to the agent being determined and chemically inert to the environments in which they are positioned. Also, if both electrodes are made from the same metal, an additional means is required for imposing a voltage across and causing direct electrical current to flow between the electrodes, thereby providing the driving force for the electrochemical reaction of the instrument. If the electrodes are made of dissimilar metals, an additional power source is not necessarily required since a galvanic couple will be formed between the electrodes. However, an external power source may be used if desired. For example, an external power source may be used in the case where the galvanic potential is insufficient as a driving force for the electrochemical reaction to provide an accurate readout for the instrument or where it is desired to decrease the rate of the electrochemical reaction, thereby increasing the effective range of the instrument.

It is an object of this invention to provide a compact, sensitive instrument for the determination of chemical oxidizing and reducing agents. It is a further object to provide apparatus which is characterized by

2

0 096 117

a relatively short time of recovery between tests. Other objects of the invention will be apparent to those skilled in the art from the more detailed description which follows.

Figure 1 is a cross-sectional view of a detector constructed according to the principles of the present invention.

Figure 2 is a partial cross-sectional view of a detector illustrating a modification of the detector shown in Figure 1 according to the principles of the present invention.

Figures 3 and 4 illustrate typical response curves for detectors constructed according to the principles of the present invention.

The following description illustrates the manner in which the principles of the invention are applied, but it is not to be construed as in any way limiting the scope of the invention.

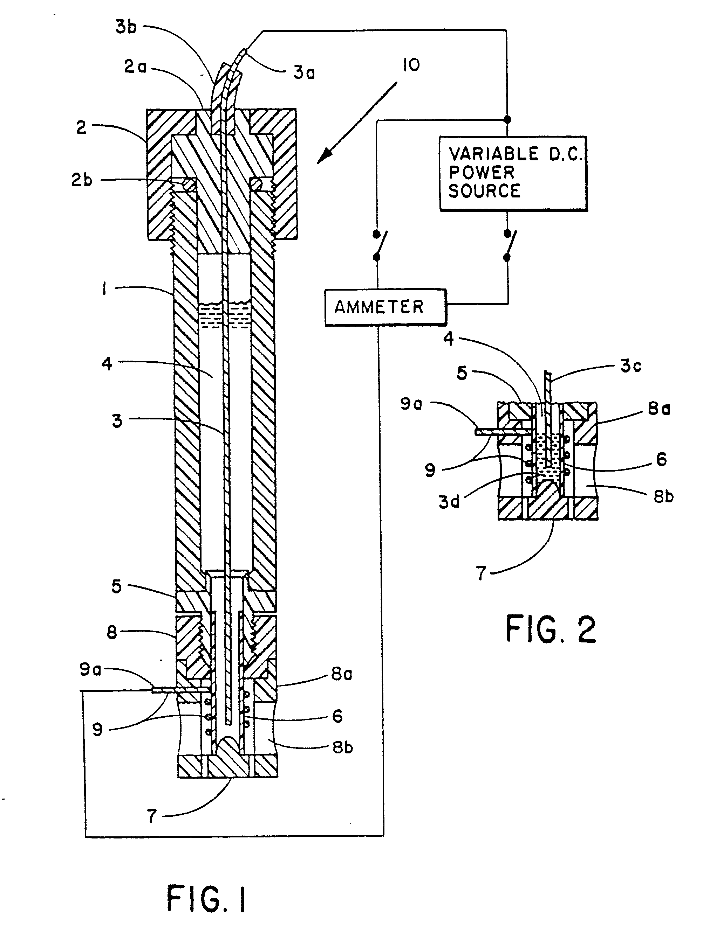

More specifically, referring to Figure 1, a detector or sensor 10 is illustrated. The sensor 10 comprises a hollow cylindrical body 1 adapted to contain an electrolyte solution 4; a means for sealing the body 1 at the top which includes a threaded cap 2, an electrically-insulating member 2a, and an "O" ring 2b. The body 1 is provided with sealing means at the bottom which includes a swagelock fitting 5, a tube 6 formed from an ion-exchange membrane material, and an end plug 7 fitted into and sealing the end of the tube 6. A nut 8 is threaded onto the swagelock fitting 5. A lower cap 8a for protecting the tube 6 is secured to the nut 8 and includes openings 8b. An electrode 3 is in contact with the electrolyte solution 4 and extends into the tube 6. The electrode 3 has a terminal end connection 3a which is covered with an electrical insulation 3b. An electrode 9 is wound around tube 6 and has a terminal end connection 9a.

Referring to Figure 2, the modified version of the sensor 10 includes a mercury pool electrode 3d in electrical contact with an electrical-lead wire 3c. All other elements of sensor 10 remain unchanged.

The electrolyte solution 4 is generally a solution of an inorganic halide. Preferably, the electrolyte solution 4 is a saturated aqueous solution of calcium chloride or lithium chloride. In addition, the electrode 3 is preferably a silver wire and electrode 9 is preferably a platinum wire when the electrodes are made from dissimilar metals. When the electrodes are made from the same metal, both electrodes 3 and 9 are preferably made of platinum. In the modified version of the sensor 10 shown in Figure 2, the electrical-lead wire 3c in contact with the mercury pool electrode 3d is preferably made of platinum. The tube 6 can be formed from any known ion-exchange membrane material depending on the requirements of the sensor 10. Preferably, if a cation-exchange membrane is desired, the tube 6 is beneficially made from a material having a polytetrafluoroethylene backbone and perfluorinated two-carbon sulfonated sidechains such as that marketed by E. I. du Pont de Nemours and Company, Inc., under the tradename of "Nafion". If an anion-exchange membrane is desired, the tube 6 is beneficially made from a styrene-divinyl-benzene copolymer having quaternary-ammonium sidechains. The remaining structural elements of the sensor 10 can be constructed of any known structural material which will also provide electrical insulation for the electrochemical circuit. For example, a plastic material such as chlorinated polyvinylchloride may beneficially be used.

To complete the analyzer, any known means (not shown) for accurately measuring the flow of electrical current is connected between the terminal end connections 3a and 9a of the sensor 10. For example, a microammeter or a resistor in parallel combination with a voltmeter may beneficially be used. A suitable measuring device includes a ten-thousand ohm resistor in parallel combination with a Hickok LX-303 Digital Voltmeter. If a source of voltage and direct electric current is imposed on the electrodes, this source may be any well-known means such as a battery or an alternating power source which has been stepped down with a direct current transformer or rectifier. A chart recorder may also be beneficially used in combination with the current measuring means to provide a permanent and continuous record. The current measuring means is also preferably adapted to read out directly in the quantity of the chemical oxidizing or reducing agent detected in the test environment.

A particularly preferred use of the sensor 10 is for measuring the concentration of a halogen such as chlorine in air. Before using a new sensor 10 for measuring the chlorine content in air, it has been found that it is preferable to pre-condition the sensor 10 to improve its sensitivity. Pre-conditioning is accomplished by making a direct electrical connection between the terminal end connections 3a and 9a, and then exposing the electrode 9 to an atmosphere of substantially pure wet chlorine gas for a period of ten to fifteen minutes. The exact reason that the sensitivity of the sensor 10 is improved by pre-conditioning is not known, but it is theorized that pre-conditioning brings a new sensor 10 to a faster chemical equilibrium across the electrochemical couple of the electrodes 3 and 9, the electrolyte solution 4 and the ion-exchange tube 6.

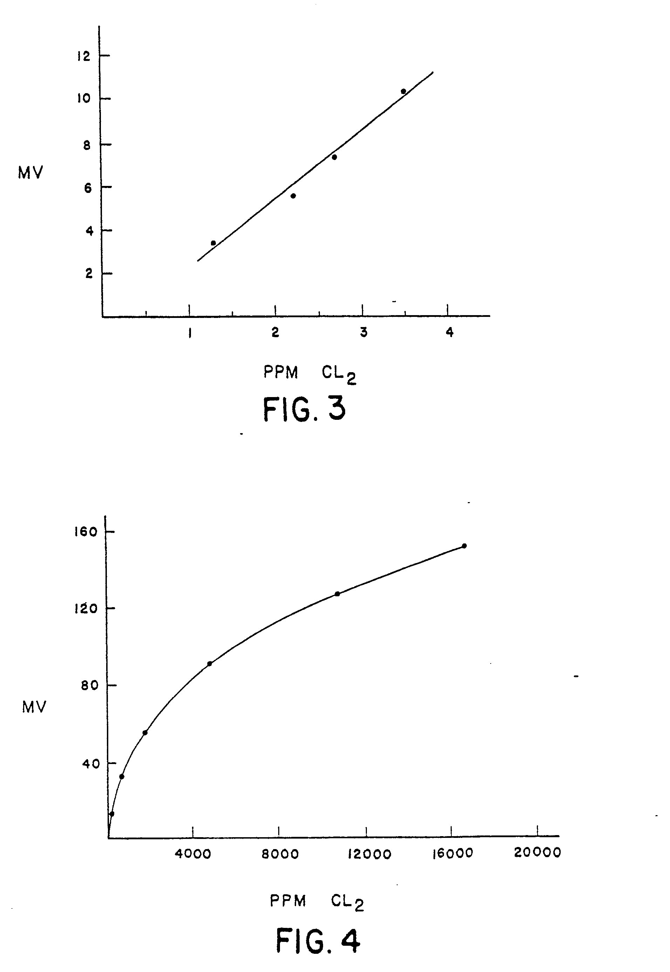

To further illustrate the present invention, a series of four test runs were made with the present sensor to determine the chlorine concentration (expressed as parts per million) in air. In each run the chlorine concentration was known and the readout in millivolts was reproducibly determined. The results of the runs are shown in the following Table 1, and are further illustrated by representative curves for Run No. 1 and Run No. 4 shown in Figures 3 and 4, respectively. In Runs 1, 2 and 3, the current-measuring device was a ten-thousand ohm resistor in parallel combination with a Hickok XL-303 Digital Voltmeter. In Run No. 4, the current-measuring device was a four-hundred and fifty ohm resistor in parallel combination with a voltmeter/stripchart recorder. In Run No. 1, a sensor, as illustrated by Figure 2, including platinum and mercury pool electrodes and a saturated calcium chloride electrolyte solution, was used. In Run No. 2, a sensor, as illustrated by Figure 1, including platinum and silver electrodes and a saturated lithium chloride

3

electrolyte solution, was used. In Run No. 3 and Run No. 4, a sensor, as illustrated by Figure 1, including platinum and silver electrodes and a saturated calcium chloride electrolyte solution, was used. In all four runs, a cation-exchange membrane tube used which was made from Nafion®.

TABLE 1

Millivolts

| $Cl_2$—PPM | Run #1 | Run #2 | Run #3 | Run #4 |
|---|---|---|---|---|
| 1.3 | 3.4 | 14.3 | 6.3 | — |
| 2.2 | 5.5 | 16.8 | 7.6 | — |
| 2.7 | 7.3 | 20.7 | 9.8 | — |
| 3.5 | 10.2 | 25.3 | 13.6 | — |
| 158 | — | — | — | 13.9 |
| 644 | — | — | — | 30.3 |
| 1,856 | — | — | — | 56.0 |
| 4,876 | — | — | — | 90.8 |
| 10,860 | — | — | — | 128.0 |
| 16,773 | — | — | — | 152.0 |

The instrument of the present invention can be modified by employing a non-aqueous electrolyte solution for determining chlorine leaks in chlorine liquefaction processes. The electrolyte here employed in an organic gel comprising (1) from 50 to 81 weight percent of a solvent such as, for example, propylene carbonate, (2) a gelling agent from 18 to 49 weight percent such as, for example, polyacrylonitrile and (3) from 0.09 to 1.1 weight percent of a salt capable of lowering the humidity in the gas that can be absorbed on the membrane. The salt preferably is lithium chloride. Optionally, the organic gel includes from 0.2 to 1.0 weight percent of a doping agent such as, for example, $KPF_6$ (potassium hexafluorophosphate). A preferred composition comprises (1) from 70 to 81 weight percent, most preferably about 81 weight percent, propylene carbonate; (2) from 18 to 29 weight percent, most preferably about 18 weight percent, polyacrylonitrile; (3) about 0.5 weight percent $KPF_6$ and (4) about 0.3 weight percent lithium chloride. Organic electrolytes for use in gas detectors are generally known from U.S. Patent 4,049,503, issued to W. J. Becker et al. on September 20, 1977.

The gelled electrolyte composition of this invention is a composition gelled with polyacrylonitrile to decrease the vapor pressure of the solvent, which increases the life span by a significant factor over other liquid based electrolytes. No loss of sensitivity has been detected in a linear detection range of from 0.04 to 5 ppm at an average response time of about six seconds for a detection level of 5 ppm. Regeneration of the instrument can be obtained by washing with ethanol.

**Claims**

1. An instrument (10) for quantitatively determining the concentration of chemical oxidizing or reducing agents in a gaseous environment, comprising a first electrode (9) exposed to said gaseous environment, a second electrode (3) in contact with an electrolyte solution (4), and means for measuring a flow of electrical current between said first (9) and said second electrode (3), characterized in that an ion-exchange membrane (6) adapted to contain said electrolyte solution is present, said ion-exchange membrane (6) separating said electrolyte solution (4) and second electrode (3) from said first electrode (9), said first electrode (9) being in electrical and physical contact with said ion-exchange membrane (6).

2. The instrument of claim 1, characterized in that said first and second electrodes (9, 3) are formed from dissimilar metals.

3. The instrument of claim 1, characterized in that said first and second electrodes (9, 3) are formed from the same metal, and said instrument (10) includes a means for imposing a voltage across and flow of direct electrical current between said first and second electrodes (9, 3).

4. The instrument of claim 2, characterized in that said ion-exchange membrane (6) is a cation-exchange membrane, whereby said instrument (10) is adapted to quantitatively determine the concentration of a chemical oxidizing agent in the gaseous environment.

4

5. The instrument of claim 4, characterized in that said cation-exchange membrane (6) is formed from a material having a polytetrafluoroethylene backbone and perfluorinated two-carbon sulfonated sidechains.

6. The instrument of claim 2 or 3, characterized in that said ion-exchange membrane (6) is an anion-exchange membrane, whereby said instrument (10) is adapted to quantitatively determine the concentration of a chemical reducing agent in the gaseous environment.

7. The instrument of claim 6, characterized in that said anion-exchange membrane (6) is formed from a styrenedivinylbenzene copolymer having quaternary-ammonium sidechains.

8. The instrument of any one of the preceding claims characterized by including means for imposing a voltage across and flow of direct electrical current between said first and second electrodes (9, 3).

9. The instrument of any one of the preceding claims characterized in that said electrolyte solution (4) is a non-aqueous solution.

10. The instrument of claim 9, characterized in that said non-aqueous electrolyte solution (4) is an organic gel comprising 1) from 50 to 81 weight percent of a solvent, 2) from 18 to 49 weight percent of a gelling agent, and 3) from 0.09 to 1.1 weight percent of a salt capable of lowering the humidity in the gas that can be absorbed on the membrane (6).

11. The instrument of claim 10, characterized in that said organic gel comprises 1) about 81 weight percent of a propylene carbonate solvent, 2) about 18 weight percent polyacrylonitrile, 3) about 0.3 weight percent lithium chloride, and 4) about 0.5 weight percent potassium hexafluorophosphate.

**Patentansprüche**

1. Vorrichtung (10) zur quantitativen Bestimmung der Konzentration von chemisch oxidierenden oder reduzierenden Mitteln in gasförmiger Umgebung, enthaltend eine erste Elektrode (9), die der gasförmigen Umgebung ausgesetzt ist, eine zweite Elektrode (3), die in Kontakt mit einer Elektrolytlösung (4) steht und Einrichtungen zum Messen eines zwischen der ersten (9) und der zweiten Elektrode (3) fließenden elektrischen Stromes, dadurch gekennzeichnet, daß eine Ionenaustauschermembrane (6) vorhanden ist, die so ausgebildet ist, daß sie den Elektrolyten enthält, die Ionenaustauschermembrane (6) die Elektrolytlösung (4) und die zweite Elektrode (3) von der ersten Elektrode (9) trennt, die erste Elektrode (9) in elektrischem und physikalischem Kontakt mit der Ionenaustauschermembrane (6) steht.

2. Vorrichtung nach Anspruch 1, dadurch gekennzeichnet, daß die ersten und zweiten Elektroden (9, 3) aus unterschiedlichen Metalen sind.

3. Vorrichtung nach Anspruch 1, dadurch gekennzeichnet, daß die ersten und zweiten Elektroden (9, 3) aus dem gleichen Metall sind und die Vorrichtung (10) Einrichtungen enthält zum Anlegen einer Spannung an die Elektroden, so daß ein direkter elektrischer Strom zwischen den ersten und zweiten Elektroden (9, 3) fließt.

4. Vorrichtung nach Anspruch 2, dadurch gekennzeichnet, daß die Ionenaustauschermembrane (6) eine Kationenaustauschermembrane ist und die Vorrichtung (10) zur quantitativen Bestimmung der Konzentration eines chemisch oxidierenden Mittels in der gasförmigen Umgebung ausgebildet ist.

5. Vorrichtung nach Anspruch 4, dadurch gekennzeichnet, daß die Kationenaustauschermembrane (6) aus einem Material ist, das eine Polytetrafluorethylen-Grundkette und perfluorierte zwei Kohlenstoffatome enthaltende sulfonierte Seitenketten aufweist.

6. Vorrichtung nach Anspruch 1 oder 2, dadurch gekennzeichnet, daß die Ionenaustauschermembrane (6) eine Anionenaustauschermembrane ist, wobei die Vorrichtung (10) zur quantitativen Bestimmung der Konzentration eines chemischen Reduktionsmittels in gasförmiger Umgebund ausgebildet ist.

7. Vorrichtung nach Anspruch 6, dadurch gekennzeichnet, daß die Anionenaustauschermembrane (6) aus Styroldivinylbenzol-Copolymer mit quartären Ammoniumgruppen aufweisenden Seitenketten ist.

8. Vorrichtung nach jedem der voranstehenden Ansprüche, dadurch gekennzeichnet, daß sie Einrichtungen zum Anlegen einer Spannung an die Elektroden aufweist, so daß ein direkter elektrischer Strom zwischen den ersten und zweiten Elektroden (9, 3) fließt.

9. Vorrichtung nach jedem der voranstehenden Ansprüche, dadurch gekennzeichnet, daß die Elektrolytlösung (4) eine nicht wässerige Lösung ist.

10. Vorrichtung nach Anspruch 9, dadurch gekennzeichnet, daß die nicht wässerige Elektrolytlösung (4) ein organisches Gel ist, das 1) von 50 bis 81 Gew.% eines Lösungsmittels, 2) von 18 bis 49 Gew.% eines Geliermittels und 3) von 0,09 bis 1,1 Gew.% eines Salzes enthält, das die Feuchtigkeit des Gases so verringert, daß es von der Membrane (6) absorbiert werden kann.

11. Vorrichtung nach Anspruch 10, dadurch gekennzeichnet, daß das organische Gel enthält 1) etwa 81 Gew.% eines Propylencarbonat-Lösungsmittels, 2) etwa 18 Gew.% Polyacrylnitril, 3) etwa 0,3 Gew.% Lithiumchlorid und 4) etwa 0,5 Gew.% Kaliumhexafluorphosphat.

**Revendications**

1. Instrument (10) pour déterminer quantitativement la concentration d'agents chimiques oxydants ou réducteurs dans une ambiance gazeuse, comportant une première électrode (9) exposée à ladite ambiance gazeuse, une seconde électrode (3) en contact avec une solution d'électrolyte (4) et des moyens pour mesurer un passage de courant électrique entre ladite première (9) et ladite seconde (3) électrode, caractérisé en ce qu'est présente une membrane échangeuse d'ions (6) prévue pour contenir ladite solution d'électrolyte, ladite membrane échangeuse d'ions (6) séparant ladite solution d'électrolyte (4) et la seconde électrode (3) d'avec ladite première électrode (9), ladite première électrode (9) étant en contact électrique et physique avec ladite membrane échangeuse d'ions (6).

2. Instrument selon la revendication 1, caractérisé en ce que ladite première électrode et ladite seconde électrode (9, 3) sont fabriquées en métaux différents.

3. Instrument selon la revendication 1, caractérisé en ce que ladite première électrode et ladite second électrode (9, 3) sont fabriquées dans le même métal; et en ce que ledit instrument (10) comporte un moyen pour appliquer une différence de potentiel et pour faire passer un courant électrique continu entre ladite première électrode et ladite seconde électrode (9, 3).

4. Instrument selon la revendication 2, caractérisé en ce que ladite membrane échangeuse d'ions (6) est une membrane échangeuse de cations, ce par quoi ledit instrument (10) est adapté à déterminer quantitativement la concentration d'un agent chimique oxydant dans une ambiance gazeuse.

5. Instrument selon la revendication 4, caractérisé en ce que ladite membrane échangeuse de cations (6) est fabriquée en un matériau présentant comme ossature un polytétrafluoroéthylène et des chaines latérales perfluorées sulfonées à deux carbones.

6. Instrument selon la revendication 2 ou 3, caractérisé en ce que ladite membrane échangeuse d'ions est une membrane échangeuse d'anions, ce par quoi ledit instrument (10) est adapté à déterminer quantitativement la concentration d'un agent chimique réducteur dans l'ambiance gazeuse.

7. Instrument selon la revendication 6, caractérisé en ce que ladite membrane échangeuse d'anions (6) est réalisée en un copolymère du styrène-divinylbenzène à chaines latérales d'ammonium quaternaire.

8. Instrument selon l'une quelconque des revendications précédentes, caractérisé en ce qu'il comporte un moyen pour appliquer une différence de potentiel et pour faire passer un courant électrique continu entre ladite première électrode et ladite seconde électrode (9, 3).

9. Instrument selon l'une quelconque des revendications précédentes, caractérisé en ce que ladite solution d'électrolyte (4) est une solution non aqueuse.

10. Instrument selon la revendication 9, caractérisé en ce que ladite solution non aqueuse d'électrolyte (4) est un gel organique comportant 1) de 50 à 81 pour cent en poids d'un solvant, 2) de 18 à 49 pour cent en poids d'un agent gélifiant, et 3) de 0.09 à 1.1 pour cent en poids d'un sel capable d'abaisser l'humidité qui se trouve dans le gaz qui peut être absorbé sur la membrane (6).

11. Instrument selon la revendication 10, caractérisé en ce que ledit gel organique comporte 1) environ 81 pour cent en poids d'un solvant carbonate de propylène, 2) environ 18 pour cent en poids de polyacrylonitrile, 3) environ 0.3 pour cent en poids de chlorure de lithium, et 4) environ 0.5 pour cent en poids d'hexafluorophosphate de potassium.

FIG. 1

FIG. 2

VARIABLE D.C.
POWER
SOURCE

AMMETER

FIG. 3

FIG. 4